# EUROPEAN PATENT SPECIFICATION

(11) **EP 1 224 173 B1**
(45) Date of publication and mention of the grant of the patent: **12.10.2005**
(21) Application number: 00982694.2
(22) Date of filing: 18.10.2000
(51) Int. Cl.: A61K 31/415, C07D 231/14

(54) **PYRAZOLE DERIVATIVES AS CANNABINOID RECEPTOR ANTAGONISTS**
PYRAZOL-DERIVATE ALS CANNABINOIDREZEPTOR-ANTAGONISTEN
DERIVES DE PYRAZOLE COMME ANTAGONISTES DES RECEPTEURS DES CANNABINOIDES

(30) Priority: 18.10.1999 US 159993 P
(43) Date of publication of application: 24.07.2002
(73) Proprietor: The University of Connecticut, Farmington, CT 06032 (US)
(72) Inventor: MAKRIYANNIS, Alexandros, Willimantic, CT 06226 (US); LIU, Qian, Storrs, CT 06268 (US)
(74) Representative: Beacham, Annabel Rose
(86) International application number: PCT/US2000/041239
(87) International publication number: WO 2001/029007

(56) References cited:
- US-A- 5 462 960
- US-A- 5 624 941
- US-A- 5 925 768
- US-A- 6 127 399
- HOWLETT, ALLYN C. ET AL: "Azido- and isothiocyanato-substituted aryl pyrazoles bind covalently to the CB1 cannabinoid receptor and impair signal transduction" JOURNAL OF NEUROCHEMISTRY (2000), 74(5), 2174-2181 , XP001097394

## Description

### Field of the Invention

The present invention relates generally to pyrazole derivatives and is more particularly concerned with new and improved pyrazole derivatives exhibiting high binding affinities for cannabinoid receptors, pharmaceutical preparations employing these analogs and these derivatives for use in therapy.

### Background of the Invention

Classical cannabinoids such as the marijuana derived cannabinoid Δ⁹-tetrahydrocannabinol (Δ⁹-THC), as well as endogenous ligands (anandamide) produce their pharmacological effects via their agonist properties at specific cannabinoid receptors in the body. So far, two cannabinoid receptors have been characterized: CB1, a central receptor found in the mammalian brain and peripheral tissues and CB2, a peripheral receptor found only in the peripheral tissues. Compounds that are agonists or antagonists for one or both of these receptors have been shown to provide a variety of pharmacological effects. See, for example, Pertwee, R.G., Pharmacology of cannabinoid CB1 and CB2 receptors, Pharmacol. Ther., (1997) 74:129 - 180 and Di Marzo, V., Melck, D., Bisogno, T., DePetrocellis, L., Endocannabinoids: endogenous cannabinoid receptor ligands with neuromodulatory action, Trends Neurosci. (1998) 21:521 - 528.

Over the last few years, a number of potent synthetic cannabinoid agonists have been developed. These agonist materials have helped in the characterization of cannabinoid receptors and with studies of receptor molecular properties.

Cannabinoid antagonists are compounds that bind to one of the CB 1 or CB2 receptors but have no effect. There is considerable interest in developing cannabinoid antagonists possessing high affinity for one of the CB1 or CB2 receptors. Such cannabinoid antagonist materials provide a tool to better understand the mechanisms by which cannabinoid agonists produce their pharmacological effects and for the development of new therapeutic agents.

One class of cannabimimetic antagonists encompasses pyrazole derivatives. Pyrazole analogs have been found to act as antagonists for the CB1 and CB2 receptors, and occasionally to act as agonists for the CB1 and CB2 receptors. Most of the known materials show high receptor affinity for only the CB1 cannabinoid receptor. See for instance, Barth, F. et al, Pyrazole Derivatives, Method Of Preparing Them And Pharmaceutical Compositions In Which They Are Present; U.S. Patent No. 5,624,941 to Barth et al, issued April 29, 1997; Rinaldi-Carmona, M. et al, SR141716A, A Potent And Selective Antagonist Of The Brain Cannabinoid Receptor, *FEBS Lett.* 1994, 350, 240-244; Rinaldi-Carmona, M. et al, Biochemical And Pharmacological Characterization Of SR141716A, The First Potent And Selective Brain Cannahinoid Receptor Antagonist, *Life Sci*. 1995, 56, 1941-1947; and Makriyannis, A., Structure-Activity Relationships Of Pyrazole Derivatives As Cannabinoid Receptor Antagonists, *J. Med. Chem.* 42, 769 - 776, 1999.

### Summary of the Invention

The invention includes several novel pyrazole derivatives and physiologically acceptable salts thereof. The invention includes materials selective for either the CB1 or CB2 receptors. Further, some of the analogs have agonistic or antagonistic properties. Pyrazole can be represented by the formula:

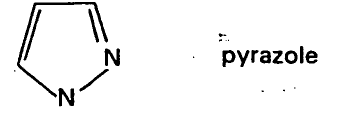

In one aspect of the invention, modifications were made to the pyrazole structure in the 1, 3, 4 and 5 position of the pyrazole ring. The novel pyrazole derivatives can generally be shown by structural formula 1.

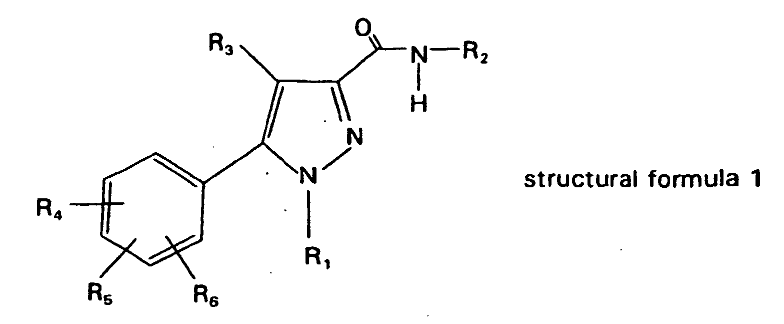

In formula 1, R₁ is a chain having the structure (CH₂)ₙZ where n is an integer from 1 to about 10 and Z is selected from the group consisting of H, halogen, N₃, NCS (isothiocyanate), CN, OH, OCH₃, NH₂ and CH =CH₂.

R₃ is selected from the group consisting of H and a chain having the structure (CH₂)ₙCH₃ where n is an integer from O to 3.

R₄, R₅ and R₆ are each independently selected from the group consisting of H, halogen, N₃, NCS, OCH₃, CH₃, CH₂CH₃, NO₂, NH₂, phenyl and phenyl with at least one substituent selected from the group consisting of halogen, N₃, NCS, OCH₃, CH₃, CH₂CH₃, NO₂, and NH₂.

R₂ is selected from the group consisting of naphthyl, 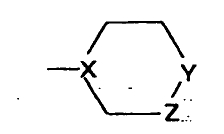 where X is selected from the group consisting of N and CH, and Y and Z are each independently selected from the group consisting of O, N, S and (CH₂)ₙ where n is an integer from 1 to about 7, 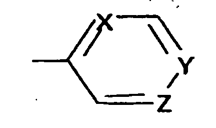 where X, Y and Z are each independently selected from the group consisting of N and CH, 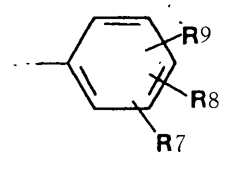 where R7, R8 and R9 are each independently selected from the group consisting of halogen, N₃, NCS, OCH₃, CH₃, CH₂CH₃, NO₂, NH₂ and phenyl; and 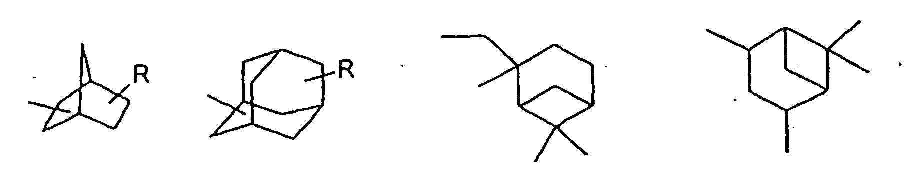 where R is selected from the group consisting of H, halogen, N₃, NCS, CN, OH, OCH₃, NH₂ and CH = CH₂.

The novel pyrazole derivatives surprisingly show high binding affinities for either or both of the CB1 and CB2 cannabinoid receptors. Some of the novel pyrazole analogs are cannabinoid receptor antagonists that prevent binding of endogenous agonists to the cannabinoid receptors and thereby block the biological actions of such endogenous agonists. Other novel analogs are cannabinoid receptor agonists. Therefore, the inventive analogs described herein, and physiologically acceptable salts thereof, have high potential when administered in therapeutically effective amounts for providing a physiological effect useful to treat pain, peripheral pain, glaucoma, epilepsy, nausea such as associated with cancer chemotherapy, AIDS Wasting Syndrome, cancer, neurodegenerative diseases including Multiple Sclerosis, Parkinson's Disease, Huntington's Chorea and Alzheimer's Disease, mental disorders such as Schizophrenia and depression; to suppress appetite; to reduce fertility; to prevent or reduce diseases associated with motor function such as Tourette's syndrome; to prevent or reduce inflammation; to provide neuroprotection; to modulate the immune system; to produce vasoconstriction or vasodilation and to effect memory enhancement. Thus, a therapeutically effective amount of an inventive compound, or a physiologically acceptable salt thereof, may be administered to an individual or animal to provide a physiological effect.

Additionally, some of the novel pyrazole derivatives have functional mo ieties such as halogen, azide and isothiocyanate and are potentially useful diagnostic agents *in vivo* (PET, SPECT). Other novel pyrazole derivatives are radioligands that are potentially useful experimental tools for cannabinoid receptor studies.

### Description of Some Preferred Embodiments

As used herein a "therapeutically effective amount" of a compound, is the quantity of a compound which, when administered to an individual or animal, results in a sufficiently high level of that compound in the individual or animal to cause a discernible increase or decrease in stimulation of cannabinoid receptors. Physiological effects that result from cannabinoid receptor stimulation include analgesia, decreased nausea resulting from chemotherapy, sedation and increased appetite. Other physiological functions include relieving intraocular pressure in glaucoma patients and suppression of the immune system. Typically, a "therapeutically effective amount" of the compound ranges from about 10 mg/day to about 1,000 mg/day.

As used herein, an "individual" refers to a human. An "animal" refers to, for example, veterinary animals, such as dogs, cats, horses and the like, and farm animals, such as cows, pigs and the like.

The compound of the present invention can be administered by a variety of known methods, including orally, rectally, or by parenteral routes (e.g., intramuscular, intravenous, subcutaneous, nasal or topical). The form in which the compounds are administered will be determined by the route of administration. Such forms include, but are not limited to, capsular and tablet formulations (for oral and rectal administration), liquid formulations (for oral, intravenous, intramuscular or subcutaneous administration) and slow releasing microcarriers (for rectal, intramuscular or intravenous administration). The formulations can also contain a physiologically acceptable vehicle and optional adjuvants, flavorings, colorants and preservatives. Suitable physiologically to acceptable vehicles may include, for example, saline, sterile water, Ringer's solution, and isotonic sodium chloride solutions. The specific dosage level of active ingredient will depend upon a number of factors, including, for example, biological activity of the particular preparation, age, body weight, sex and general health of the individual being treated.

The inventive pyrazole derivatives can generally be described with reference to structural Formula 1: 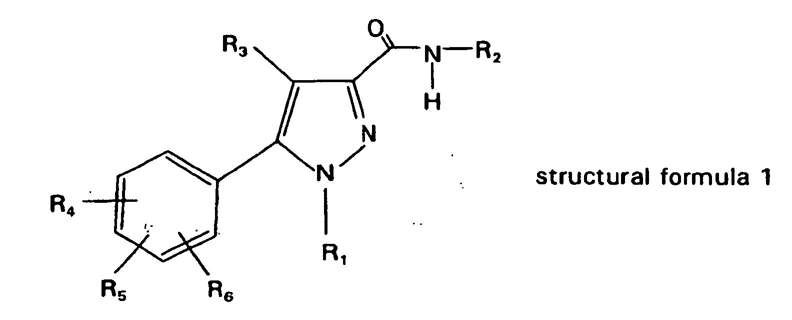 and physiologically acceptable salts thereof. With reference to structural formula 1, R₁ is a chain having the structure (CH₂)ₙZ where n is an integer from 1 to about 10 and Z is selected from the group consisting of H, halogen, N₃, NCS, CN, OH, OCH₃, NH₂ and CH = CH₂.

R₃ is selected from the group consisting of H and a chain having the structure (CH₂)ₙCH₃ where n is an integer from 0 to 3.

R₄, R₅ and R₆ are each independently selected from the group consisting of H, halogen, N₃, NCS, OCH₃, CH₃, CH₂CH₃, NO₂, NH₂, phenyl and phenyl with at least one substituent from the group consisting of halogen, N₃, NCS, OCH₃, CH₃, CH₂CH₃, NO₂, and NH₂.

R₂ is selected from the group consisting of naphthyl, 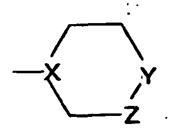 where X is selected from the group consisting of N and CH, and Y and Z are each independently selected from the group consisting of O, N, S and (CH₂)ₙ where n is an integer from 1 to about 7, 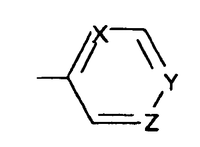 where X, Y and Z are each independently selected from the group consisting of N and CH, 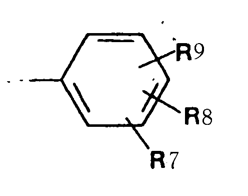 where R7, R8 and R9 are each independently selected from the group consisting of halogen, N₃, NCS, OCH₃, CH₃, CH₂CH₃, NO₂, NH₂ and phenyl; and 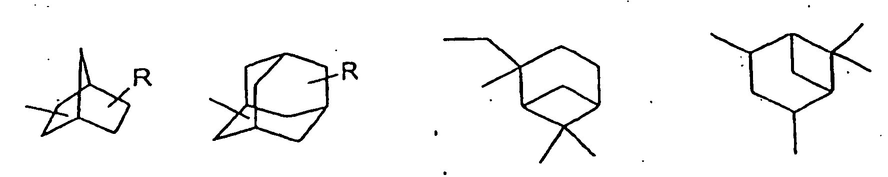 where R is selected from the group consisting of H, halogen, N₃, NCS, CN, OH, OCH₃, NH₂ and CH = CH₂.

The following examples are given for purposes of illustration only in order that the present invention may be more fully understood. These examples are not intended to limit in any way the practice of the invention. The above materials were prepared as follows. The prepared cannabimimetic pyrazole derivatives can generally be described with reference to the structures of TABLE 1 below. Naturally, the novel pyrazole derivatives are intended to include physiologically acceptable salts thereof.

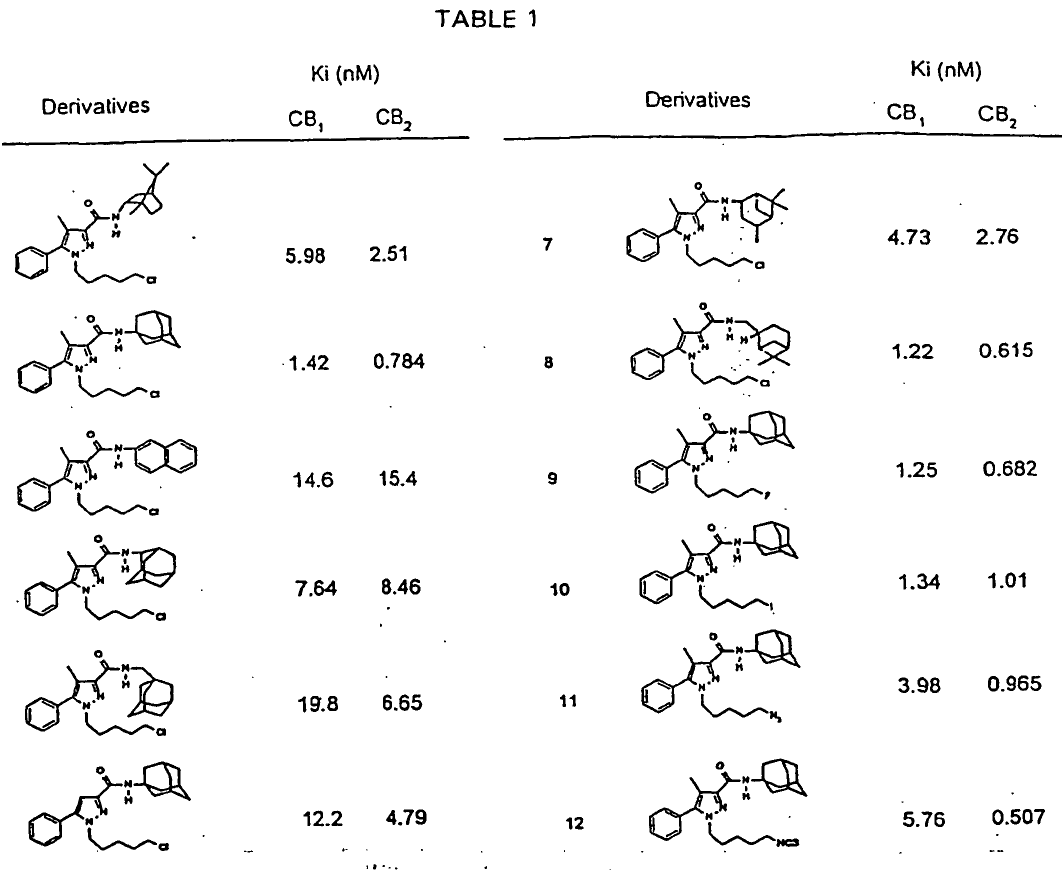

**General**. Flash column chromatography was carried out using Whatman active silica gel (230 - 400 mesh) and eluents were distilled before use. Solvents for reactions were dried or purified as required. Reactions were carried out under nitrogen atmospheres unless otherwise noted.

### General procedure for the preparation of compound 1-5 and 7-8:

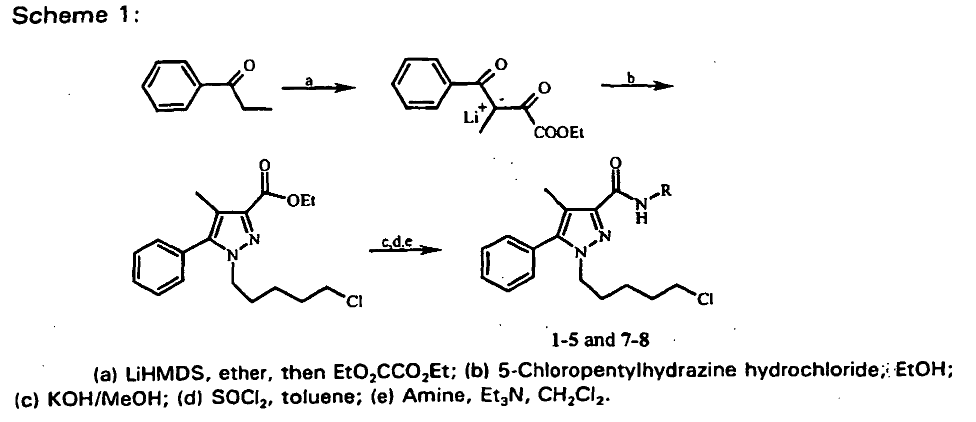

**Lithium salt of ethyl 2,4-dioxo-3-methyl-4-phenylbutanoate.** To a magnetically stirred solution of lithium bis(trimethylsilyl)amide (40 ml, 1.0 M solution in hexane, 40 mmol) in diethyl ether (120 mL) was added a solution of propiophenone (5.37 g, 40 mmol) in diethyl ether (50 mL) at 78 °C. The mixture was stirred at the same temperature for an additional 45 min, after which diethyl oxalate (6.4 mL, 47 mmol) was added to the mixture. The reaction mixture was allowed to warm to room temperature and stirred for 16 hours (h). The precipitate was filtered, washed with diethyl ether, and dried under vacuum to afford the lithium salt (7.78 g, 83% yield).

**1-(5-Chloropentyl)-4-methyl-5-phenyl-1*****H*****-pyrazole-3-carboxylic acid, Ethyl Ester.** To a magnetically stirred solution of the above lithium salt (2.0 mmol) in 10 mL of ethanol was added a solution of 5-chloropentylhydrazine hydrochloride (2.2 mmol) at room temperature. The resulting mixture was stirred at room temperature for 20 h. The solvent was removed under reduced pressure and the residue was partitioned between ethyl acetate (20 mL) and water (10 mL). The water phase was extracted with ethyl acetate (2x, 15 mL each). The ethyl acetate solution was washed with brine, dried over anhydrous sodium sulfate, filtered, and evaporated. The crude product was purified by flash column chromatography on silica gel with a petroleum ether/ethyl acetate mixture to afford the ester as a colorless oil.

**Compounds 1-5 and 7-8**. To a magnetically stirred solution of the above ethyl ester (3.4 mmol) in methanol (15 mL) was added a solution of potassium hydroxide (8.6 mmol) in methanol (12 mL). The mixture was heated under reflux for 3hours. The cooling reaction mixture was then poured into 10 mL of water and acidified with 10% hydrochloric acid. The precipitate was filtered, washed with water, and dried under vacuum to yield the corresponding acid (1.4g, 100% yield) as a white solid.

A solution of the crude acid (3.4 mmol) and thionyl chloride (10.3 mmol) in toluene (15 mL) was refluxed for 3hours. The solvent was evaporated under reduced pressure. The residue was then redissolved in 40 ml of toluene and evaporated to yield the crude carboxylic chloride as an oil.

A solution of the carboxylic chloride (31.5 mmol) in dichloromethane (160 mL) was added dropwise to a solution of an appropriate amine (47.2 mmol) and triethylamine (6.5 mL, 46.7 mmol) in dichloromethane (90 mL) at 0°C. After stirring at room temperature for 3 h, brine was added to the reaction mixture, which was extracted with dichloromethane (3x, 200 mL each). The combined extracts were washed with brine, dried over anhydrous sodium sulfate, filtered, and evaporated. Flash column chromatography on silica gel with a petroleum ether/acetone (4:1) mixture gave carboxamide **1-5** and **7-8**.

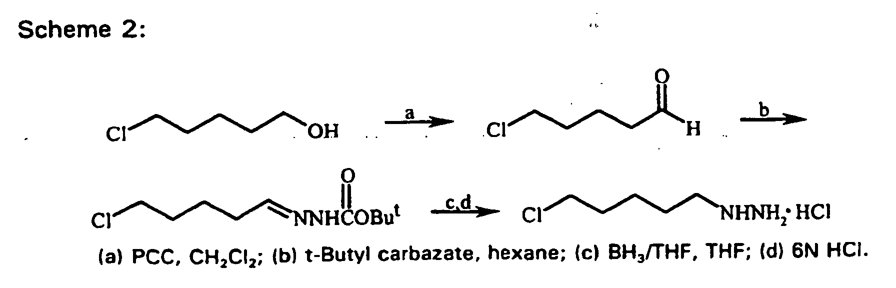

**5-Chloropentylhydrazine hydrochloride.** A hexane solution of 5-chloropentylaldehyde (10.0 mmol) and *tert*-butyl carbazate (1.32 g, 10.0 mmol) was refluxed for 20 min. After cooling to the room temperature, the crystallized *tert*-butyl carbazate derivative was collected by filtration and dried in vacuum. A 1.0 M solution of borane tetrahydrofuran complex in tetrahydrofuran (10.0 mL, 10.0 mmol) was added to the solid *tert-*butyl carbazate derivative (10.0 mmol), the resulting mixture was allowed to stir at room temperature for 10 min, and then 6N hydrochloric acid (5.0 mL) was added dropwise. The reaction mixture was refluxed for 10 min and evaporated to dryness under reduced pressure. Tetrahydrofuran was added to the residue, after which boric acid was removed by filtration. After removal of the solvent under reduced pressure, the residue was crystallized from a solution of tetrahydrofuran and diethyl ether to give 5-chloropentylhydrazine as its hydrochloride salt (71% yield).

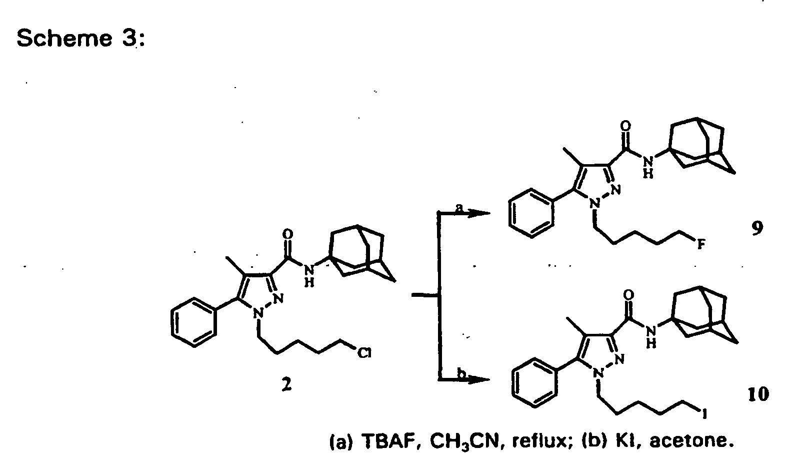

### Preparation of compound 9:

To a magnetically stirred solution of compound **2** (0.40 g, 0.91 mmol) in acetonitrile (15 mL) was added a 1.O M solution of tetrabutylammonium fluoride (4.5 mL, 4.5 mmol) in tetrahydrofuran and the mixture was refluxed overnight. The reaction mixture was then quenched by saturated aqueous ammonium chloride and extracted with diethyl ether (3x, 50 mL each). The combined extracts were washed with brine, dried over anhydrous sodium sulfate, filtered and evaporated. Purification by flash column chromatography on silica gel with a petroleum ether/acetone (9:1) mixture gave compound **9** as a white solid (0.296 g, 77% yield).

### Preparation of compound 10:

To a magnetically stirred solution of compound 2 (1.12 g, 2.6 mmol) in acetone (25 mL) was added sodium iodide (1.72g, 11.5 mmol). The reaction mixture was refluxed for 25 hours and evaporated to dryness under reduced pressure. The residue was partitioned between diethyl ether (100 mL) and water (40 mL), and the water phase was extracted with diethyl ether (3x, 30 mL each). The combined extracts were washed with brine, dried over anhydrous sodium sulfate, filtered, and evaporated. Purification by flash column chromatography on silica gel with a methylene chloride/acetone (30:1) mixture gave compound **10** as a white solid (1.27 g, 94 % yield).

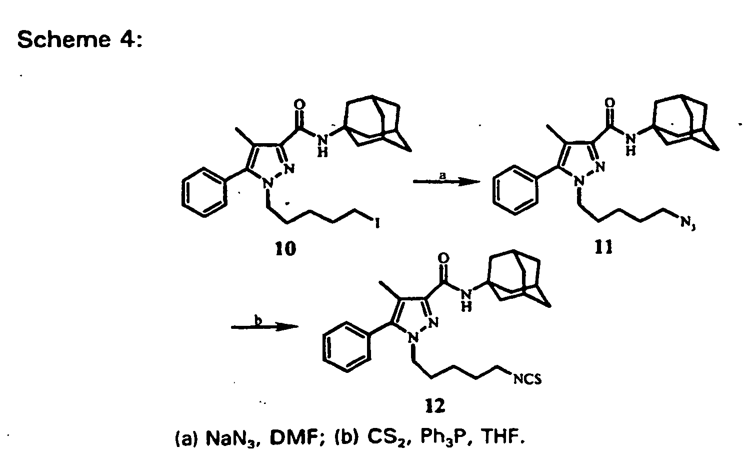

### Preparation of compound 11:

To a magnetically stirred solution of compound **10** (0.675 g, 1.3 mmol) in anhydrous N,N-dimethylformamide (17 mL) was added sodium azide (0.83 g, 12.7 mmol). The resulting mixture was stirred at room temperature for 40 hours. Brine was then added and the reaction mixture was extracted with diethyl ether (3x, 20 mL each). The combined extracts were washed with brine, dried over anhydrous sodium sulfate, filtered and evaporated. The residue was purified by flash column chromatography on silica gel with a methylene dichloride/acetone (50:1) mixture to afford compound **11** as a white solid (0.203 g, 35.8 % yield).

### Preparation of compound 12:

To a magnetically stirred solution of compound **11** (0.359 g, 0.8 mmol) in tetrahydrofuran (5 mL) was added triphenylphosphine (0.32 g, 1.22 mmol), followed by carbon disulfide (1.44 mL, 24 mmol). The reaction mixture was stirred at room temperature for 70 hours and then evaporated under reduced pressure. The residue was purified by flash column chromatography on silica gel with methylene dichloride to afford compound **12** (0.288 g, 77.6% yield).

### Preparation of compound 6:

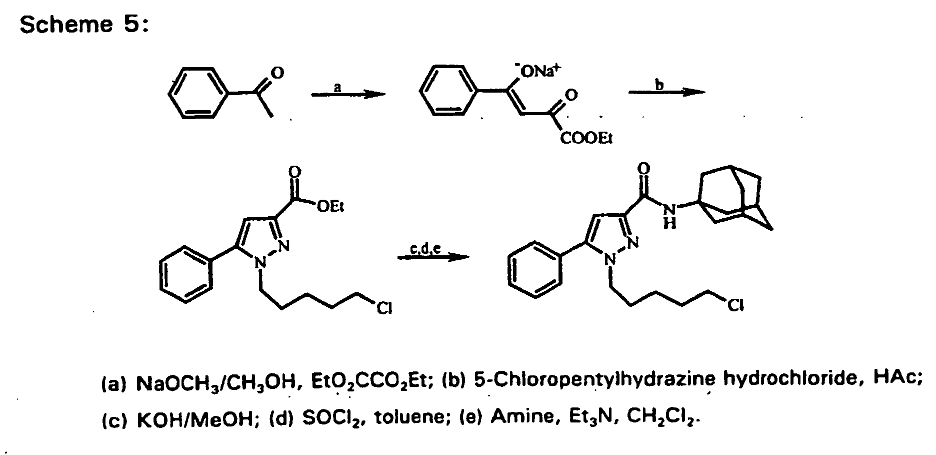

**Sodium salt of methyl benzoylpyruvate.** 1.3 g of sodium was dissolved in 25 mL of anhydrous methanol. A mixture of 5.8 mL of acetophenone and 6.7 mL of diethyl oxalate in 60 mL of methanol was then added, the temperature being kept below 10 °C. The reaction mixture was then stirred at room temperature for 3 hours, after which 100 mL of dry ether was added. Stirring was continued for 20 min, the mixture was filtered and precipitate was washed with ether and dried under vacuum to give 6.32 g of the expected sodium salt.

**1-(5-Chloropentyl)-5-phenyl-1*****H*****-pyrazole-3-carboxylic acid; Methyl Ester.** A suspension of 0.605 g of the sodium salt obtained above and 0.502 g of 5-chloropentylhydrazine hydrochloride in 6.5 mL of acetic acid was refluxed for 4 hours. After cooling, the mixture was poured on to 6.5 g of ice and the crystals obtained were filtered off, washed with water and dried under vacuum to give 0.42 g of ester.

**Compound 6.** Compound 6 was prepared from the methyl ester according to the procedure described for the compound 1-5 and 7-8.

The materials were tested for CB2 receptor binding affinity and for CB1 receptor affinity (to determine selectivity for the CB2 receptor). As used herein, "binding affinity" is represented by the IC₅₀ value which is the concentration of an analog required to occupy 50% of the total number (Bmax) of the receptors. The lower the IC₅₀ value, the higher the binding affinity. As used herein an analog is said to have "binding selectivity" if it has higher binding affinity for one receptor compared to the other receptor; e.g. a cannabinoid analog which has an IC₅₀ of 0.1 nM for CB1 and 10 nM for CB2, is 100 times more selective for the CB1 receptor. The binding affinities (Kᵢ) are expressed in nanomoles (nM) and are listed in TABLE 1.

For the CB1 receptor binding studies, membranes were prepared from rat forebrain membranes according to the procedure of P.R. Dodd et al, A Rapid Method for Preparing Synaptosomes: Comparison with Alternative Procedures, Brain Res., 107 - 118 (1981). The binding of the novel analogues to the CB1 cannabinoid receptor was assessed as described in W.A. Devane et al, Determination and Characterization of a Cannabinoid Receptor in a Rat Brain, Mol. Pharmacol., 34, 605 - 613 (1988) and A. Charalambous et al, 5'-azido Δ⁸ THC: A Novel Photoaffinity Label for the Cannabinoid Receptor, J. Med. Chem., 35, 3076 - 3079 (1992) with the following changes. The above articles are incorporated by reference herein.

Membranes, previously frozen at -80°C, were thawed on ice. To the stirred suspension was added three volumes of TME (25mM Tris-HCl buffer, 5 mM MgCl₂ and 1 mM EDTA) at pH 7.4. The suspension was incubated at 4°C for 30 min. At the end of the incubation, the membranes were pelleted and washed three times with TME.

The treated membranes were subsequently used in the binding assay described below. Approximately 30 µg of membranes were incubated in silanized 96-well microtiter plate with TME containing 0.1% essentially fatty acid-free bovine serum albumin (BSA), 0.8 nM [³H] CP-55,940, and various concentrations of test materials at 30 °C for 1 hour. The samples were filtered using Packard Filtermate 196 and Whatman GF/C filterplates and washed with wash buffer (TME containing 0.5% BSA). Radioactivity was detected using MicroScint 20 scintillation cocktail added directly to the dried filterplates, and the filterplates were counted using a Packard Instruments Top-Count. Nonspecific binding was assessed using 100 nM CP-55,940. Data collected from three independent experiments performed with duplicate determinations was normalized between 100% and 0% specific binding for [³H] CP-55,940, determined using buffer and 100 nM CP-55,940. The normalized data was analyzed using a 4-parameter nonlinear logistic equation to yield IC₅₀ values. Data from at least two independent experiments performed in duplicate was used to calculate IC₅₀ values which were converted to Kᵢ values using the assumptions of Cheng et al, Relationship Between the Inhibition Constant (Kᵢ) and the concentration of Inhibitor which causes 50% Inhibition (IC₅₀) of an Enzymatic Reaction, Biochem. Pharmacol., 22, 3099-3102, (1973).

For the CB2 receptor binding studies, membranes were prepared from frozen mouse spleen essentially according to the procedure of P.R. Dodd et al, A Rapid Method for Preparing Synaptosomes: Comparison with Alternative Procedures, Brain Res., 226, 107 - 118 (1981) which is incorporated by reference herein. Silanized centrifuge tubes were used throughout to minimize receptor loss due to adsorption. The CB2 binding assay was conducted in the same manner as for the CB1 binding assay. The binding affinities (Kᵢ) were also expressed in nanomoles (nM).

Compound SR141716A, a known pyrazole derivative, has a cannabinoid receptor affinity (Kᵢ) of 11.5 nM for the CB1 receptor and 1640 nM for the CB2 receptor. As can be seen from the results in TABLE 1, all of the inventive compounds have receptor affinities much higher than compound SR141716A for at least one of the CB1 or CB2 receptors. In fact, most of the inventive pyrazole derivatives have receptor affinities much higher (lower numerically) than compound SR141716A for both of the CB1 and CB2 receptors.

The physiological and therapeutic advantages of the inventive materials can be seen from the above disclosure and also with additional reference to the following references, the disclosures of which are hereby incorporated by reference. Arnone M., Maruani J., Chaperon P, *et al*, Selective inhibition of sucrose and ethanol intake by SR141716, an antagonist of central cannabinoid (CB1) receptors, Psychopharmacal, (1997) 132, 104-106. Colombo G, Agabio R, Diaz G. et al: Appetite suppression and weight loss after the cannabinoid antagonist SR141716. Life Sci. (1998) 63-PL13-PL117. Simiand J, Keane M, Keane PE, Soubrie P: SR 141716, A CB1 cannabinoid receotor antagonist, selectively reduces sweet food intake in marmoset. Behav. Pharmacol (1998) 9:179-181. Brotchie JM: Adjuncts to dopamine replacement a pragmatic approach to reducing the problem of dyskinesia in Parkinson's disease. Mov. Disord. (1998) 13:871-876. Terranova J-P, Storme J-J Lafon N et al: Improvement of memory in rodents by the selective CB1 cannabinoid receptor antagonist, SR 141716. Psycho-pharmacol (1996) 126:165-172. Hampson AL Grimaldi M. Axpirod J. Wink D: Cannabidiol and (-) Δ⁹ tetrahydrocannabinol are neuroprotective antioxidants. Proc. Natl Acad Sci. USA (1998) 9S:8268-8273. Buckley NE, McCoy KI, Mpzey E et al Immunomodulation by cannabinoids is absent in mice deficient for the cannabinoid CB₂ receptor. Eur. J Pharmacol (2000) 396:141-149. Morgan Dr: Therapeutic Uses of Cannabis. Harwood Academic Publishers, Amsterdam. (1997). Joy JE, Wagtson SJ, Benson JA: Marijuana and Medicine Assessing the Science Base. National Academy Press, Washington, DC, USA (1999). Shen M. Thayer SA: Cannabinoid receptor agonists protect cultured rat hippocampal neurons from excitotoxicity. Mol. Pharmacol (1996) 54:459-462. DePetrocellis L, Melck D, Palmisano A. et al: The endogenous cannabinoid anandamide inhibits human breaast cancer cell proliferation. Proc Natl. Acad. Sci USA (1998) 95:8375-8380. Green K. Marijuana smoking vs. cannabinoids for glaucoma therapy. Arch. Ophibalmol. (1998) feb 433-1437. Hemming M, Yellowlees PM, Effective treatment of Tourette's syndrome with marijuana. J. Psychopharmacol, (1993) 7:389-391. Muller-Vahl KB, Schneider U, Kolbe H, Emrich, HM. Treatment of Tourette's syndrome with delta-9-tetrahydrocannabinol. Am. J. Psychiat. (1999) 156-195. Muller-Vahl KB, Kolbe H, Schneider U, Emrich, HM Cannabis in movement disorders. Porsch. Kompicmentarmed (1999) 6 (suppl. 3) 23-27. Consroe P, Musty R, Rein J, Tillery W, Pertwee R. The perceived effects of smoked cannabis on patents with multiple sclerosis, Eur. Neurol. (1997) 38-44-48. Pinnegan-Ling D, Musty R. Marinol and phantom limb pain: a case study. Proc Inv. Cannabinoid Rea. Sec. (1994):53. Brenneisen R, Pgli A, Elsohly MA, Henn V. Spiess Y: The effect of orally and rectally administered Δ⁹⁻ tetrahydrocannabinol on spasticity, a pilot study with 2 patients. Int. J. Clin Pharmacol Ther. (1996) 34:446-452. Martyn CN. IIIis LS, Thom J. Nabilone in the treatment of multiple sclerosis. Lancet (1995) 345:579. Maurer M, Henn V, Dittrich A, Hofmann A. Delta-9-tetrahydrocannabinol shows antispastic and analgesic effects in a single case double-blind trial. Eur. Arch. Psychiat. Clin. Neurosci. (1990), Z40:1-4. Herzberg U, Eliav E, Bennett GJ, Kopin IJ: The analgesic effects of R(+) WIN 55,212-2 mesylate, a high affinity cannabinoid agonist in a rare model of neuropathic pain. Neurosci. Letts. (1997) 221 :157-160. Richardson JD, Kilo S. Hargreaves KM, Cannabinoids reduce dryperalgesia and inflammation via interaction with peripheral CB1 receptors. Pain (1998) 75:111-119. Ricardson JD, Aanonsen I, Hargreaves KM: Antihyperalgesic effects of a spinal cannabinoids. Eur. J. Pharmacol. (1998) 346:145-153. Calignano A, La Rana G. Diuffrida A, Piomelli D: Control of pain initiation by endogenous cannabinoids. Nature (1998) 394:277-291. Wagner JA, Varga K, Jarai Z, Kunos G: Mesenteric vasodilation mediated by endothelia anandamide receptors. Hypertension (1999) 33:429-434. Schuel, H., Burkman, L.J., Picone, R.P., Bo, T., Makriyannis, A., Cannabinoid receptors in human sperm. Mol. Biol. Cell., (1997) (8), 325a.

The inventive analogs described herein, and physiologically acceptable salts thereof, have high potential when administered in therapeutically effective amounts for providing a physiological effect useful to treat pain, peripheral pain, glaucoma, epilepsy, nausea such as associated with cancer chemotherapy, AIDS Wasting Syndrome, cancer, neurodegenerative diseases including Multiple Sclerosis, Parkinson's Disease, Huntington's Chorea and Alzheimer's Disease, mental disorders such as Schizophrenia and depression; to suppress appetite; to reduce fertility; to prevent or reduce diseases associated with motor function such as Tourette's syndrome; to prevent or reduce inflammation; to provide neuroprotection; to modulate the immune system; to produce vasoconstriction or vasodilation and to effect memory enhancement. Thus, a therapeutically effective amount of an inventive compound, or a physiologically acceptable salt thereof, may be administered to an individual or animal to provide a physiological effect.

Those skilled in the art will recognize, or be able to ascertain with no more than routine experimentation, many equivalents to the specific embodiments of the invention disclosed herein. Such equivalents are intended to be encompassed by the scope of the invention.

## Claims

1. A compound of the formula 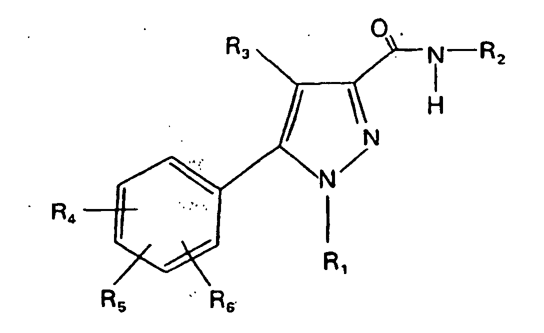 or a physiologically acceptable salt thereof, wherein:
R₁ is a chain having the structure (CH₂)ₙZ where n is an integer from 1 to 10 and Z is selected from the group consisting of H, halogen, N₃, NCS, CN, OH, OCH₃, NH₂ and CH =CH₂;
R₃ is selected from the group consisting of H and a chain having the structure (CH₂)ₙCH₃ where n is an integer from 0 to 3;
R₄, R₅ and R₆ are each independently selected from the group consisting of H, halogen, N₃, NCS, OCH₃, CH₃, CH₂CH₃, NO₂, NH₂, phenyl and phenyl with at least one substituent from the group consisting of halogen, N₃, NCS, OCH₃, CH₃, CH₂CH₃, NO₂, and NH₂; and
R₂ is selected from the group consisting of naphthyl, 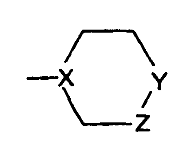
where X is selected from the group consisting of N and CH, and Y and Z are each independently selected from the group consisting of O, N, S and (CH₂)ₙ where n is an integer from 1 to 7, 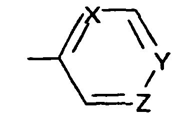 where X, Y and Z are each independently selected from the group consisting of N and CH, 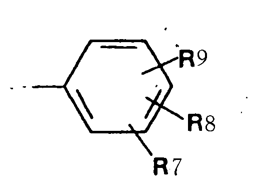 where R7, R8 and R9 are each independently selected from the group consisting of halogen, N₃, NCS, OCH₃, CH₃, CH₂CH₃, NO₂, NH₂ and phenyl; and 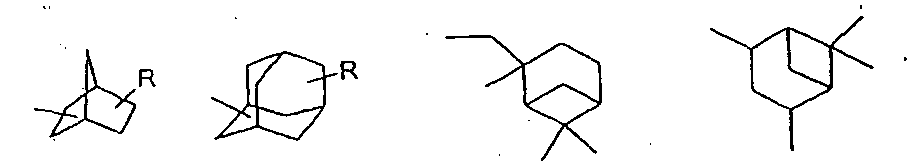 where R is selected from the group consisting of H, halogen, N₃, NCS, CN, OH, OCH₃, NH₂ and CH=CH₂.

2. A compound of the formula 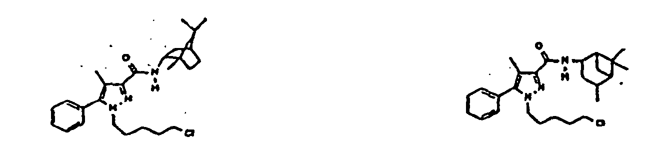 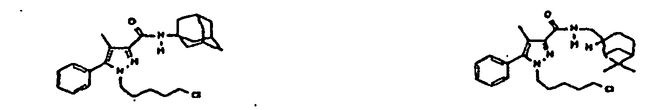 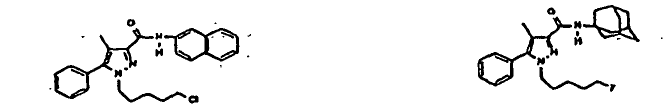 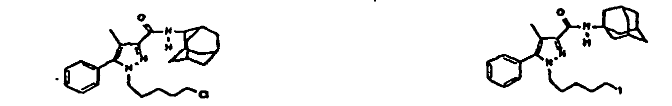 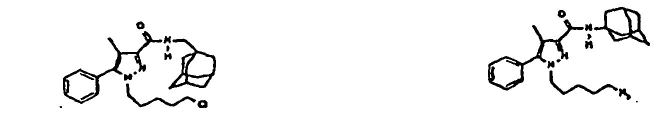 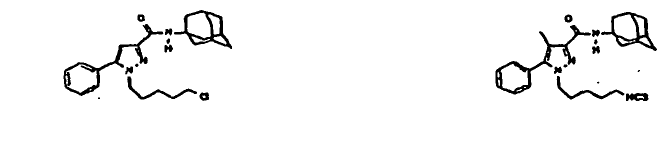 or a physiologically acceptable salt form thereof.

3. A pharmaceutical composition comprising a pharmaceutically acceptable carrier and a compound as claimed in claim 1 or claim 2.

4. A compound as claimed in claim 1 or claim 2, for use in therapy.

5. A compound as claimed in claim 1 or claim 2, for use in preferentially binding to the cannabinoid receptors in an individual or animal.

6. Use of a compound of claim 1 or claim 2, or a physiologically acceptable salt form thereof, in the manufacture of a medicament for use in the treatment of pain, peripheral pain, glaucoma, epilepsy, nausea, AIDS Wasting Syndrome, cancer, neurodegenerative diseases, mental disorders, inflammation, or Tourette's syndrome; or to suppress appetite, reduce fertility, effect memory enhancement, modulate the immune system or to provide neuroprotection.

## Patentansprüche

1. Verbindung der Formel 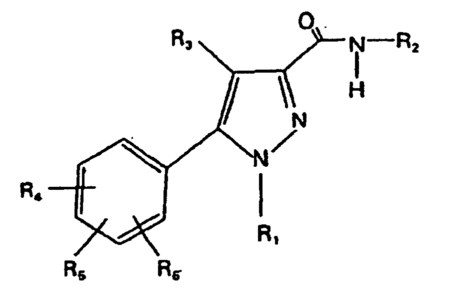 oder ein physiologisch annehmbares Salz davon, worin:
R₁ eine Kette mit der Struktur (CH₂)ₙZ ist, wobei n eine ganze Zahl von 1 bis 10 ist und Z ausgewählt ist aus der Gruppe bestehend aus H, Halogen, N₃, NCS, CN, OH, OCH₃, NH₂ und CH=CH₂;
R₃ ausgewählt ist aus der Gruppe bestehend aus H und einer Kette mit der Struktur (CH₂)ₙCH₃, wobei n eine ganze Zahl von 0 bis 3 ist;
R₄, R₅ und R₆ jeweils unabhängig ausgewählt sind aus der Gruppe bestehend aus H, Halogen, N₃, NCS, OCH₃, CH₃, CH₂CH₃, NO₂, NH₂, Phenyl und Phenyl mit wenigstens einem Substituenten aus der Gruppe bestehend aus Halogen, N₃, NCS, OCH₃, CH₃, CH₂CH₃, NO₂ und NH₂; und
R₂ ausgewählt ist aus der Gruppe bestehend aus Naphthyl, 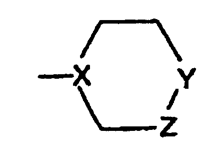 worin X ausgewählt ist aus der Gruppe bestehend aus N und CH, und Y und Z jeweils unabhängig ausgewählt sind aus der Gruppe bestehend aus O, N, S und (CH₂)ₙ, wobei n eine ganze Zahl von 1 bis 7 ist, 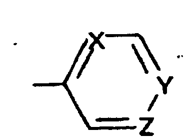 worin X, Y und Z jeweils unabhängig ausgewählt sind aus der Gruppe bestehend aus N und CH, 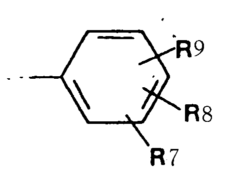 worin R₇, R₈ und R₉ jeweils unabhängig ausgewählt sind aus der Gruppe bestehend aus Halogen, N₃, NCS, OCH₃, CH₃, CH₂CH₃, NO₂, NH₂ und Phenyl; und 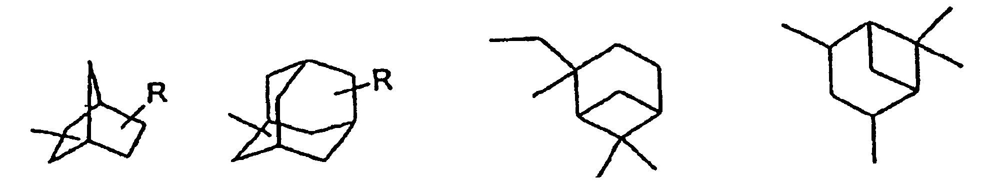 worin R ausgewählt ist aus der Gruppe bestehend aus H, Halogen, N₃, NCS, CN, OH, OCH₃, NH₂ und CH=CH₂.

2. Verbindung der Formel 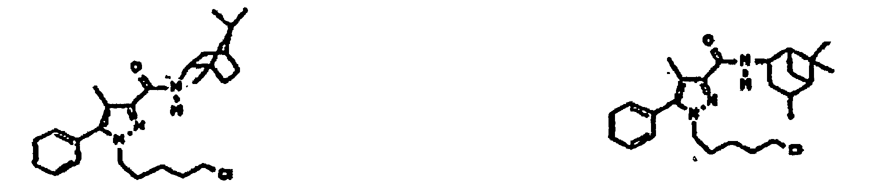 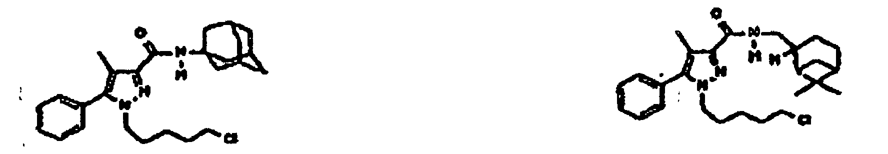 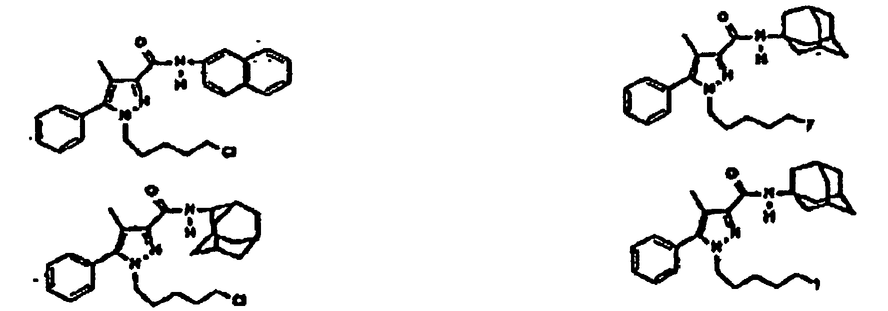 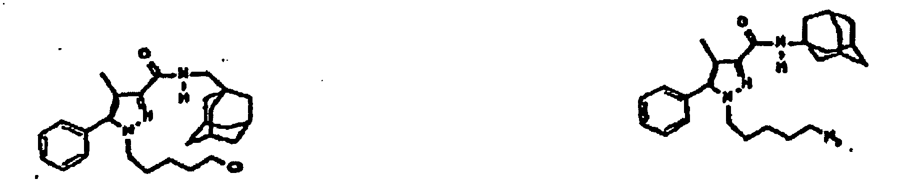 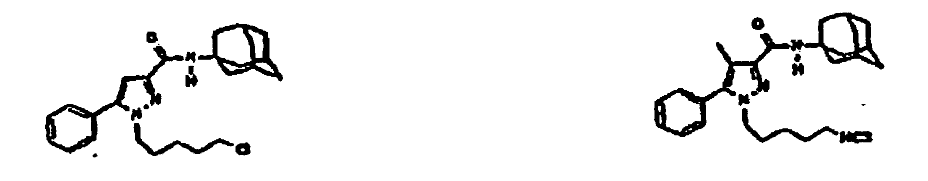 oder eine physiologisch annehmbare Salzform davon.

3. Pharmazeutische Zusammensetzung, umfassend einen pharmazeutisch annehmbaren Träger und eine Verbindung nach Anspruch 1 oder Anspruch 2.

4. Verbindung nach Anspruch 1 oder Anspruch 2 zur therapeutischen Verwendung.

5. Verbindung nach Anspruch 1 oder Anspruch 2, zur Verwendung um vorzugsweise an die Cannabinoid-Rezeptoren bei einer Person oder einem Tier zu binden.

6. Verwendung einer Verbindung nach Anspruch 1 oder Anspruch 2, oder eine physiologisch annehmbare Salzform davon, bei der Herstellung eines Medikaments zur Behandlung von Schmerz, peripherem Schmerz, Glaukom, Epilepsie, Nausea, AIDS Wasting-Syndrom, Krebs, neurodegenerativen Erkrankungen, mentalen Erkrankungen, Entzündung, Tourette-Syndrom; oder um Appetit zu zügeln, Fruchtbarkeit zu verringern, um Gedächtnissteigerung herbeizuführen, um das Immunsystem zu modulieren oder um Neuroprotektion bereitzustellen.

## Revendications

1. Composé de formule: 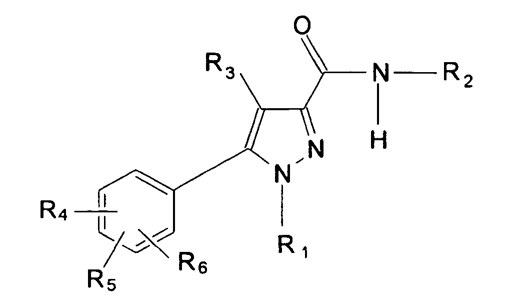 ou un sel physiologiquement acceptable de celui-ci, dans lequel :
R₁ est une chaîne ayant la formule chimique développée (CH₂)ₙZ où n est un nombre entier de 1 à 10 et Z est choisi dans le groupe constitué par un atome d'hydrogène H, un groupe halogène, N₃, NCS, CN, OH, OCH₃, NH₂ et CH=CH₂;
R₃ est choisi dans le groupe constitué par un atome d'hydrogène H et une chaîne ayant la formule chimique développée (CH₂)ₙCH₃ où n est un nombre entier de 0 à 3;
R₄, R₅ et R₆ sont choisis indépendamment chacun dans le groupe constitué par un atome d'hydrogène H, un groupe halogène, N₃, NCS, OCH₃, CH₃, CH₂CH₃, NO₂, NH₂, phényle et un groupe phényle avec au moins un substituant choisi dans le groupe constitué par un groupe halogène, N₃, NCS, OCH₃, CH₃, CH₂CH₃, NO₂, et NH₂ ; et
R₂ est choisi dans le groupe constitué par un groupe naphtyle, 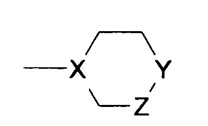 où X est choisi dans le groupe constitué par un atome d'azote N et un groupe CH, et Y et Z sont choisis indépendamment chacun dans le groupe constitué par un atome d'oxygène 0, un atome d'azote N, un atome de soufre S et un groupe (CH₂)ₙ où n est un nombre entier de 1 à 7, 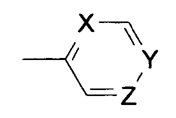 où X, Y et Z sont choisis indépendamment chacun dans le groupe constitué par un atome d'azote N et un groupe CH, 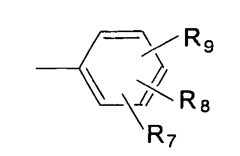 où R₇, R₈ et R₉ sont choisis indépendamment chacun dans le groupe constitué par un groupe halogène, N₃, NCS, OCH₃, CH₃, CH₂CH₃, NO₂, NH₂ et phényle ; et 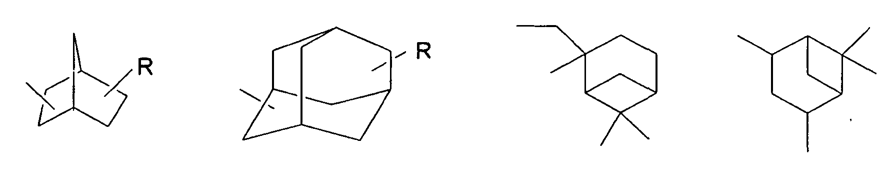 où R est choisi dans le groupe constitué par un atome d'hydrogène H, un groupe halogène, N₃, NCS, CN, OH, OCH₃, NH₂ et CH=CH₂.

2. Composé de formule : 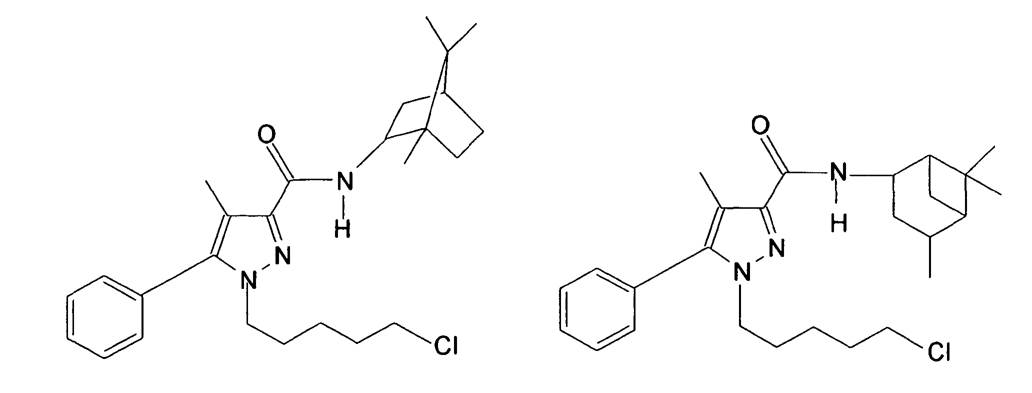 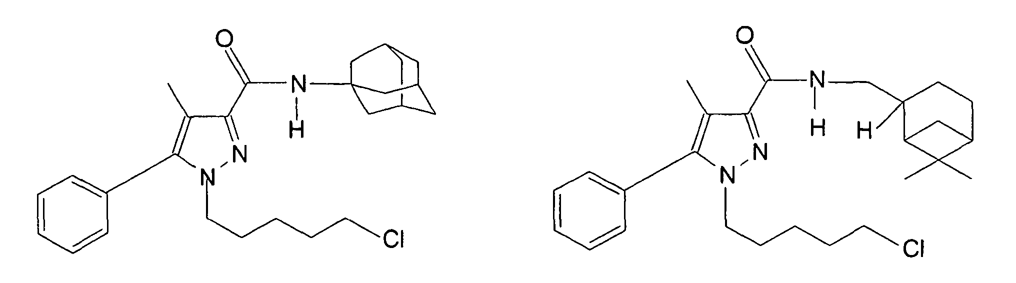 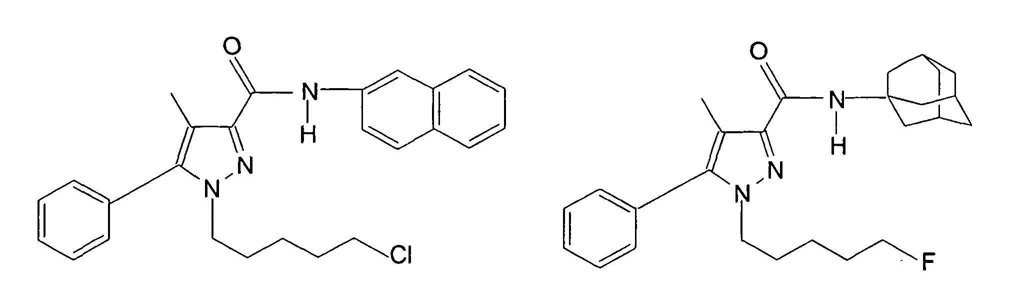 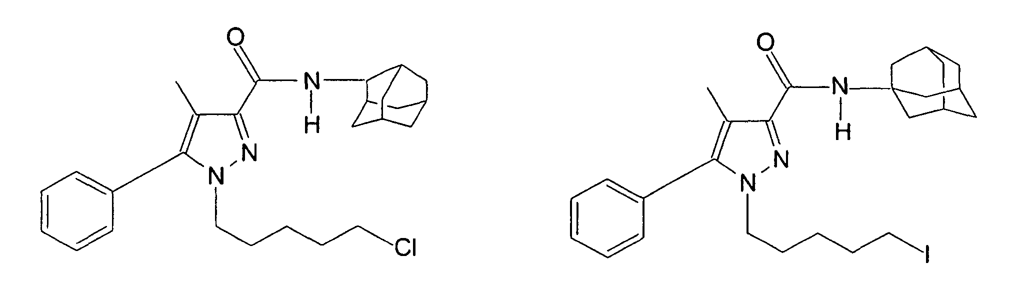 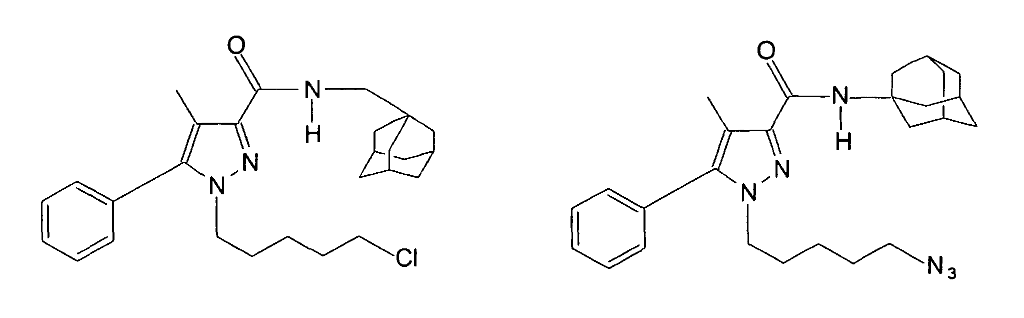 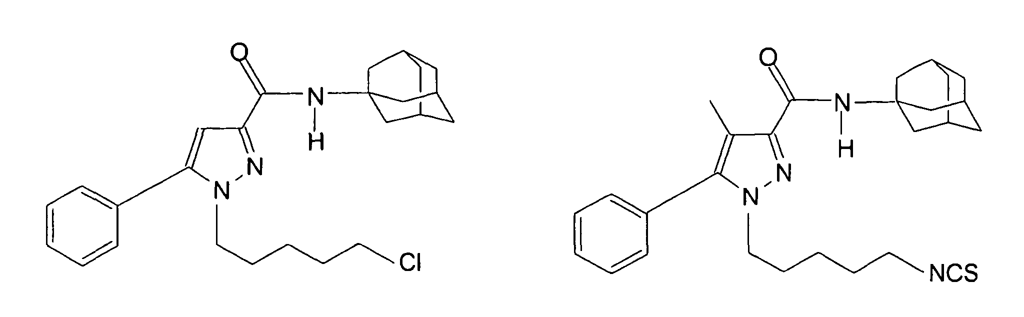 ou une forme saline physiologiquement acceptable de celui-ci.

3. Composition pharmaceutique comprenant un support pharmaceutiquement acceptable et un composé tel que revendiqué selon la revendication 1 ou la revendication 2.

4. Composé selon la revendication 1 ou la revendication 2, pour une utilisation en thérapie.

5. Composé selon la revendication 1 ou la revendication 2, pour une utilisation dans la liaison sélective aux récepteurs des cannabinoïdes chez un individu ou un animal.

6. Utilisation d'un composé selon la revendication 1 ou la revendication 2, ou d'une forme saline physiologiquement acceptable de celui-ci, dans la fabrication d'un médicament pour une utilisation dans le traitement de la douleur, de la douleur périphérique, du glaucome, de l'épilepsie, de la nausée, de l'atrophie musculaire causée par le SIDA, du cancer, des maladies neurodégénératives, des troubles mentaux, de l'inflammation ou de la maladie de Gilles de la Tourette; ou pour supprimer l'appétit, réduire la fertilité, apporter une amélioration de la mémoire, moduler le système immunitaire ou pour fournir une neuroprotection.
